# EUROPÄISCHE PATENTANMELDUNG

(11) **EP 1 561 746 A2**
(43) Veröffentlichungstag der Anmeldung: **10.08.2005**
(21) Anmeldenummer: 05001281.4
(22) Anmeldetag: 22.01.2005
(51) Int. Cl.: C07C 263/10, C07C 263/20, C08G 18/76, C08G 18/10, C09J 175/04

(54) **Verfahren zur Herstellung von hochreinem 2,4 '-Methylendiphenyldiisocyanat**

(30) Priorität: 04.02.2004 DE 102004005319
(71) Anmelder: Bayer MaterialScience AG, 51368 Leverkusen (DE)
(72) Erfinder: Pirkl, Hans-Georg, Dr., 51377 Leverkusen (DE); Bolton, Jeffrey, Dr., Baytown, TX 77520 (US); Meckel, Walter, Dr., 40627 Düsseldorf (DE); Wolf, Ulrich, 47647 Kerken (DE); Wintermantel, Matthias, Dr., 51061 Köln (DE); Mahrenholtz, Jochen, 47802 Krefeld (DE)

(57) **Zusammenfassung**

Die Erfindung betrifft ein Verfahren zur Herstellung von 2,4'-MDI haltigen MDI-Fraktionen, bei dem der 2,2'-MDI-Anteil aus der MDA-Herstellung aus dem Isomerengemisch weitgehend entfernt wird. Auf dieser Basis lassen sich hochreaktive monomere MDI-Produkte hergestellen, die bei ihrer Verarbeitung durch deutlich verminderte Emissionen an MDI-Monomeren gekennzeichnet sind.

## Beschreibung

Die Erfindung betrifft ein Verfahren zur Herstellung von Diisocyanaten der Diphenylmethanreihe mit sehr hohen Gehalten an 2,4'-Methylendiphenyldiisocyanat sowie ihre Verwendung zur Herstellung von Prepolymeren und Polymeren.

Aromatische Isocyanate sind sehr wichtige Rohstoffe für die Herstellung von Polyurethan-Werkstoffen. Hierbei spielen die Di- und Polyisocyanate der Diphenylmethanreihe (MDI) die mengenmäßig größte Rolle.

Unter Polyisocyanaten der Diphenylmethanreihe werden Isocyanate und Isocyanatgemische folgenden Typs verstanden: 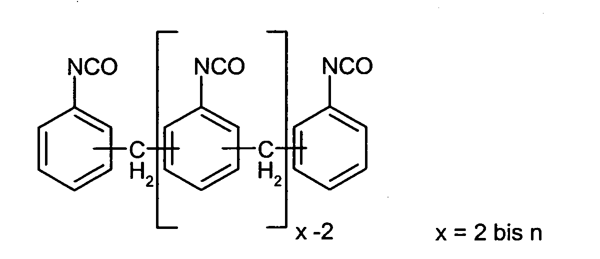 wobei n für eine natürliche Zahl > 2 steht.

Analog werden unter Polyaminen der Diphenylmethanreihe Verbindungen und Verbindungsgemische folgenden Typs verstanden: 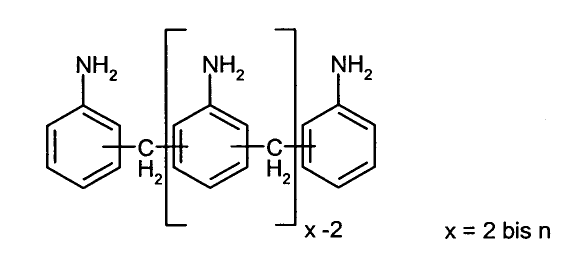 Es ist bekannt, dass Di- und Polyisocyanate der Diphenylmethanreihe (MDI) durch Phosgenierung der entsprechenden Di- und Polyamine der Diphenylmethanreihe (MDA) hergestellt werden. Die Di- und Polyamine der Diphenylmethanreihe selbst werden durch Kondensation von Anilin und Formaldehyd hergestellt. Durch Phosgenierung der Diamine der Diphenylmethanreihe erhält man die entsprechenden Diisocyanate 2,2'-MDI, 2,4'-MDI und 4,4'-MDI, die in der Fachwelt als 2-Kern-MDI (Diisocyanate der Diphenylmethanreihe) beschrieben werden. Während der Kondensation von Anilin und Formaldehyd reagiert das 2-Kern-MDA (Methylendiphenyldiamin) jedoch auch noch weiter mit Formaldehyd und Anilin zu höherkernigen MDA-Typen, die nach der Phosgenierung den Mehrkemgehalt im polymeren MDI (Polyisocyanate der Diphenylmethanreihe) darstellen.

Das bei der Phosgenierung erzeugte rohe MDI-Gemisch kann in der Polymer- / Monomer - Trennung mittels einfacher Verdampfung oder Destillation in 2-Kern-MDI (monomeres MDI) und eine Polymer-MDI-Fraktion aufgetrennt werden. Das Isomerengemisch der 2-Kern-MDI-Fraktion enthält dabei neben den Diisocyanaten 2,2'-MDI, 2,4'-MDI und 4,4'-MDI auch noch Nebenkomponenten wie Lösungsmittelrückstände oder Phenylisocyanat-Derivate. Die monomere 2-Kern-MDI-Fraktion wird nach dem Stand der Technik destillativ oder per Kristallisation in die Isomeren 4,4'-MDI und eine Mischung von etwa 50 % 2,4'-MDI und 50 % 4,4'-MDI zerlegt. Die beiden monomeren Produkte werden als Polyurthan-Rohstoff auf dem Weltmarkt angeboten oder zu Mischprodukten mit Polymer-MDI weiterverarbeitet.

Kommerziell ist hochreines 2,4'-MDI in großtechnischen Mengen zur Zeit nicht verfügbar obwohl viele positive Eigenschaften von 2,4'-MDI inzwischen bekannt sind. So kann beispielsweise in Polyurethan-Weichformschaumsystemen ein 2,4'-MDI das übliche TDI-System aus 2,4- und 2,6-TDI ersetzen (EP-B1-06 76 434). Auch in hitzehärtbaren 1-K Polyurethan-Systemen konnte 2,4'-MDI erfolgreich eingesetzt werden (EP-B1-04 31 331).

Während das 4,4'-Isomere durch seine hohe Reaktivität und der ausgesprochen guten Ausbildung von Hartsegmenten das wichtigste Isomere darstellt, zeichnet sich das 2,4'-Isomere wegen seiner unterschiedlichen Reaktivität insbesondere für den Einsatz von sehr niederviskosen, relativ Monomer-armen Prepolymeren aus.

So werden in der WO-A-93/09158 wenig Monomer enthaltende Prepolymere beschrieben, die bei NCO/OH-Verhältnissen von 1,6 bis 1,8 und Polyethern mit sekundären Hydroxylgruppen bei Verwendung von unterschiedlich reaktiven NCO-Gruppen im Molekül zu Monomer-armen Prepolymeren führen. Monomeres MDI mit einem Gehalt von 92 Gew.-% 2,4'-Diisocyanatodiphenylmethan wird als ein Beispiel angeführt. Bedeutende Einsatzgebiete sind Klebstoffe und Beschichtungen, insbesondere Folienverbundsysteme mit niedrigen Migrationswerten.

Eine Anwendung für Diphenylmethandiisocyanate ist die Herstellung von Folienverbunden, die im Bereich der Lebensmittelverpackung eingesetzt werden. Die hygienisch einwandfreie, eine hohe Lagerstabilität garantierende, preiswerte Verpackung von Lebensmitteln wird heute mit Verbundfolien erreicht, wobei durch Verkleben von Folien mit unterschiedlichen Barriereeigenschaften der Verbund optimal auf die jeweiligen Erfordernisse zugeschnitten werden kann. Polyurethane stellen die Klebstoffe der Wahl dar. Diese werden in Lösemittel-haltiger und Lösemittel-freier Form angewandt. Durch den Trend zu Lösemittel-freien Systemen haben sich mehr und mehr 2-Komponentensysteme durchgesetzt, die aus einer Polyolmischung und einem Prepolymeren auf Basis von Isocyanaten bestehen. Wegen den auf den Folienoberflächen adsorbierten Feuchtigkeitsspuren hat es sich als notwendig erwiesen, dass die Klebstoffe mit einem relativ hohen Überschuss von Isocyanatgruppen gegenüber den OH-Gruppen eingesetzt werden müssen.

Dieser hohe Überschuss an Isocyanaten stellt aber auch einen limitierenden Faktor in der weiteren Verarbeitung der Folienverbunde dar, da die Folienverbunde frei von aromatischen Aminen sein müssen ehe sie in Kontakt mit Lebensmitteln treten dürfen.

Bevor die Verbundfolien weiterverarbeitet werden können müssen sie daher gelagert werden bis keine Amine mehr nachweisbar sind. Diese Zeit ist abhängig von sehr vielen Faktoren, wie beispielsweise Eigenschaften des verwandten Klebstoffs, Art der Folien (Typ und Dicke), der herrschenden Temperatur und Luftfeuchtigkeit. Dabei ist die Anwesenheit von aromatischen Aminen in den Prüflebensmitteln vermutlich so zu erklären, dass monomere, nicht abreagierte Isocyanate durch die dünnen Folien migrieren, und auf der Oberfläche mit Feuchtigkeit langsam zu Polyharnstoffen abreagieren, die unter den normalen Bedingungen der Lebensmittellagerung stabil sind. Solange diese Reaktion nicht abgeschlossen ist, können anwesende monomere Isocyanate durch die im Test vorhandenen Prüflebensmittel partiell auch zu Aminen hydrolisiert werden.

Wegen der sehr viel größeren Reaktivität der 4-Positition im Vergleich zur 2-Positition der NCO-Gruppe im MDI können MDI-Isomere mit mindestens einer 4-NCO-Gruppe wesentlich schneller in ein oligomeres oder polymeres Polyurethannetzwerk integriert und so an der Migration durch die Folie gehindert werden. Hierdurch sinkt nach einer MDI-Prepolymerherstellung und bei der Verarbeitung dieses MDI-Prepolymers mit Polyolen die Konzentration an noch freien, ungebundenen monomeren MDI-Isomeren mit 4-NCO-Gruppe (4,4'- und 2,4'-MDI) extrem schnell ab. Wegen seiner deutlich geringeren Reaktivität verbleibt das 2,2'-MDI dagegen länger in monomerer Form in der Klebstoffschicht als die anderen MDI-Isomere und kann somit länger durch die Folie migrieren. Daher ist der Gehalt an 2,2'-MDI im MDI-Isomerengemisch eine kritische Größe und sollte so niedrig wie möglich liegen.

### Aus dem Stand der Technik zur Herstellung von MDI ist weiterhin folgendes bekannt:

Die Herstellung von MDI-Mischprodukten, welche die verschiedenen MDI-Isomeren enthalten, durch eine gezielte Synthese des MDA, welches die entsprechenden MDA-Isomere enthält, ist aus der Literatur bekannt. In EP-B1-158059 wird die Herstellung eines besonders hohen 2-Kern-Gehalt bei einer MDA-Mischung mit ca. 80 % 4,4'-MDA und ca. 10 % 2,4'-MDA bei einem 2-Kem-Gehalt von ca. 90 % offenbart. Dagegen kann die Ausbeute an 2,4'-MDA gezielt erhöht werden, wie dies in EP-B1-3303 mit einem MDA mit 88 Gew.-% 2-Kern-MDA mit 19 Gew.-% 2,2'-MDA, 36 Gew.-% 2,4'-MDA und 45 Gew.-% 4,4'-MDA gelehrt wird. Insbesondere die Herstellung hoch 2,4'-MDA-haltiger, monomerreicher MDA-Typen führt zu einer hohen Nebenausbeute an 2,2'-MDA. Wegen seiner Reaktionsträgheit ist das daraus gebildete 2,2'-MDI jedoch in vielen Anwendungen in hohen Konzentrationen nicht erwünscht.

Die Herstellung von hoch polymer-haltigem MDA mit 2-Kern-Gehalten von 46 bis 65 % ist beispielsweise in DE-A1-2750975 und DE-A1-2517301 beschrieben.

Die wesentlichen Parameter, mit denen der Anteil an 2,4'-MDA bei der Kondensation von Anilin und Formaldehyd eingestellt werden kann, sind bekannt. In der Regel wird der Gehalt an 2-Kern-MDA durch den Überschuss an Anilin bei der Kondensation eingestellt. Den Anteil an 2,4'-MDA im 2-Kern-MDA kann durch einen niedrigen Protonierungsgrad während der Kondensation, das heißt durch ein niedriges molares Verhältnis von HCl : Anilin von beispielsweise < 0,2 : 1, oder eine hohe Reaktionstemperatur eingestellt werden (DE-A1-34 07 494).

Die großtechnische Herstellung von Isocyanaten durch Umsetzung der entsprechenden Amine mit Phosgen in Lösungsmitteln ist bekannt und in der Literatur ausfiihrlich beschrieben (Ullmanns Enzyklopädie der technischen Chemie, 4. Auflage, Band 13, Seite 347-357, Verlag Chemie GmbH, Weinheim, 1977). Die MDA-Phosgenierung führt zunächst zum rohen MDI-Gemisch. Auch die Herstellung von monomerem MDI und polymerem MDI aus dem rohen MDI-Gemisch durch Destillation oder Kristallisation ist in der einschlägigen Literatur prinzipiell bekannt.

Aus der rohen monomeren 2-Kern-MDI-Fraktion des ursprünglichen rohen MDI-Gemisches werden nach dem heutigen Stand der Technik im Wesentlichen zwei Hauptprodukte isoliert. Das erste der beiden Hauptprodukte der Isomerentrennung sind ein 4,4'-MDI reiches Isomerengemisch ("4,4'-Produkt"), das praktisch frei von 2,2'-MDI ist und noch < 3 Gew.-% 2,4'-MDI enthält. Das zweite der beiden Hauptprodukte der Isomerentrennung ist ein 2,4'-MDI-reiches Gemisch ("2,4'/4,4'-Produkt"), das 20 bis 70 Gew.-% 2,4'-MDI, bis zu 3 Gew.-% 2,2'-MDI sowie als Rest 4,4'-MDI enthält. Zur Herstellung dieser beiden Hauptprodukte werden heute in der Regel die folgenden beiden technischen Verfahren ausgehend von der rohen monomeren 2-Kern-MDI-Fraktion, die aus dem rohen MDI-Gemisch aus der Polymer-/Monomer-Trennung erhalten wurde, eingesetzt:
a) Destillation, z.B. nach DE-A1-31 45 010 oder DE-A1-26 31 168 oder
b) Kristallisation, z.B. nach EP-A2-482 490 oder DE-A- 25 32 722.

Die Fachwelt konzentriert sich heute auf die möglichst ökonomische Herstellung der monomeren Isomerenmischungen, des "4,4'-Produkts" und des "2,4'/4,4'-Produkts" (M. Stepanski, P. Faessler: "New hybrid process for purification and separation of MDI isomers",Sulzer Chemtech, Presentation at the Polyurethanes Conference 2002 in Salt Lake City, 10/2002).

In WO-A1-02/070581 wird die Herstellung von höher konzentriertem 2,4'-MDI ausgehend von einem MDA-Gemisch mit hohen Gehalten an 2,4'-MDA, das durch Kondensation von Anilin und Formaldehyd bei niedrigem Anilin-Protonierungsgrad erhalten wurde, beschrieben. Eine Reinigung des so erhaltenen 2,4'-MDI von 2,2'-MDI ist in dem Verfahren gemäß WO-A1-02/070581 nicht vorgesehen. Gerade bei der Herstellung von 2,4'-reichen MDA-Gemischen bei einem niedrigen Protonierungsgrad, das heißt einem niedrigen Verhältnis von HCl zu Anilin, entstehen aber überproportional große Mengen an 2,2'-MDA (EP-B1-3303), die vor der Anwendung bei der Polyurethan-Herstellung zumindest partiell abgetrennt werden müssen. Tatsächlich ist die Reinheit des 2,4'-MDI bezüglich 2,2'-MDI jedoch ein wesentliches Qualitätsmerkmal, das sogar wichtiger ist als die Reinheit bezüglich 4,4'-MDI.

Das in WO-A1-02/070581 beschriebene Verfahren, in dem ein MDI-Gemisch mit hohen Gehalten an 2,4'-MDI durch Phosgenierung eines entsprechenden MDA-Gemisches mit hohen Anteilen an 2,4'-MDA hergestellt wird, entspricht der im Stand der Technik gängigen Methode zur Herstellung einer MDI-Mischung enthaltend ca. 50 Gew.-% 2,4'-MDI und ca. 50 Gew.-% 4,4'-MDI. Da es sich bei 2,4'-MDI um einen Leichtsieder im Vergleich zu 4,4'-MDI handelt, wird 2,4'-MDI destillativ als Kopfprodukt gewonnen. Wesentlich ist in diesem Fall, dass sich weitere Leichtsieder, insbesondere das 2,2'-MDI, in dem Kopfprodukt anreichern. Trennt man konventionell 4,4'-MDI von der 2,4'-Fraktion ab, so erhält man als erstes Produkt das weiter oben bereits genannte erste Hauptprodukt "4,4'-Produkt" mit zumeist etwa 1-2 Gew.-% 2,4'-MDI sowie als zweites Hauptprodukt das weiter oben bereits genannte zweite Hauptprodukt "2,4'/4,4'-Produkt", das eine Mischung von 2,4'-MDI und 4,4'-MDI in der Nähe des eutektischen Punktes darstellt. Hierbei reichert sich das 2,2'-Isomere siedepunktsbedingt im eutektischen Gemisch an. Ein typischer Gehalt an 2,2'-MDI in diesem eutektischen Gemisch liegt zwischen 0,8 und 5 Gew.-%.

Analoges geschieht bei der Gewinnung von "4,4'-Produkt" per Kristallisation. Hier konzentriert sich das 2,2'-Isomere zwangsläufig mit der 2,4'-reichen Fraktion in der Mutterlauge auf. Wird nun die erhaltene Mutterlauge in die Isomere 2,4'-MDI als Destillat und 4,4'-MDI im Sumpf einer Destillationskolonne getrennt, reichert sich das unerwünschte, reaktionsträge 2,2'-MDI ebenfalls in der gewünschten 2,4'-MDI-Fraktion an. Man erhält je nach Ausgangsqualität der rohen 2-Kern-MDI-Fraktion ein 2,4'-MDI mit 0,8 bis 5 Gew.-% 2,2'-MDI. Weiterhin können leichtsiedende Nebenkomponenten wie Phenylisocyanate und Lösungsmittelspuren in das Destillat gelangen, wenn diese nicht zunächst aus der Ausgangsmischung entfernt werden.

Wird dagegen versucht, die erhaltene Mutterlauge mit den Isomeren 2,4'-MDI und 4,4'-MDI durch Kristallisation zu trennen, so muss zunächst der eutektische Punkt mit einem Nicht-Kristallisationsverfahren überschritten werden, um nicht nur reines 4,4'-MDI-Kristallisat und eine 2,4'-haltige Mutterlauge mit hohen Gehalten an 4,4'-MDI zu erhalten. Daher kann mit einem reinen Kristallisationsverfahren ausgehend von der rohen 2-Kern-MDI-Fraktion kein hochangereichertes 2,4'-MDI hergestellt werden. Der eutektische Punkt kann beispielsweise durch ein Destillationsverfahren wie oben beschrieben überschritten werden. In diesem Fall kann das in dieser Patentanmeldung offenbarte Verfahren zur Herstellung hochreiner 2,4'-MDI-Fraktionen alternativ im Schritt d) mit einem Kristallisationsverfahren zur Abtrennung des größten Teiles an 4,4'-MDI ausgeführt werden.

In DE-A-26 31 168 wird ein Verfahren zur Herstellung von MDI-Gemischen mit niedrigen Gehalten an Rest-Chlor per Destillationsverfahren beschrieben. Nach dem in DE-A-26 31 168 beschriebenen Verfahren lassen sich so auch MDI-Gemische mit einem Gehalt an 2,4'-MDI von über 97 % und unter 50 ppm Chlor herstellen.

Der hohe Gehalt an 2,2'-MDI ist für praktisch alle Anwendungen störend, da es reaktionsträge ist und sich nur unter drastischen Reaktionsbedingungen vollständig in ein polymeres Netzwerk einbinden lässt. Zumeist verbleiben signifikante Anteile an 2,2'-MDI bei der Verarbeitung als Restmonomer zurück und werden über die Zeit möglicherweise freigesetzt oder reagieren wenig gezielt mit Luftfeuchtigkeit ab und führen zu verschlechterten Polymer-Eigenschaften. Auch Restspuren von Lösungsmitteln sind bei der Polyurethan-Herstellung unerwünscht und stören mit ihrem unangenehmen Geruch die Produktqualität. Etwa enthaltene Spuren an Phenylisocyanaten wiederum wirken in der Polyurethan-Reaktion als Kettenabbrecher und verschlechtern die Polymer-Eigenschaften.

Die Aufgabe der vorliegenden Erfindung ist daher, ein Verfahren zur Herstellung von MDI-Isomerengemischen mit hohen Gehalten an 2,4'-MDI bereit zu stellen, deren Gehalte an bei der Herstellung von Polyurethanen störenden Komponenten wie 2,2'-MDI, Lösungsmittelreste und Phenylisocyanate auf ein nicht mehr störendes Niveau abgesenkt sind.

Die Erfindung betrifft ein Verfahren zur Herstellung einer Fraktion von Diisocyanaten der Diphenylmethanreihe enthaltend mindestens 99 Gew.-% 2-Kern-Methylendiphenyldiisocyanat bezogen auf die Masse der Fraktion , bei dem man
a) Anilin und Formaldehyd in Gegenwart eines sauren Katalysators zu Di- und Polyaminen der Diphenylmethanreihe enthaltend 2-Kern-Methylendiphenyldiamin umsetzt, und
b) die Di- und Polyamine der Diphenylmethanreihe enthaltend 2-Kern-Methylendiphenyldiamin gegebenenfalls in Gegenwart eines Lösungsmittels phosgeniert, wobei man ein rohes Di- und Polyisocyanat erhält, und
c) aus dem rohen Di- und Polyisocyanat eine Fraktion enthaltend mindestens 95 Gew.-% 2-Kern-Methylendiphenyldiisocyanat mit einem Gehalt an 4,4'-MDI von 49 - 95,99 Gew.-%, einem Gehalt an 2,4'-MDI von 4 bis 45 Gew.-% und einem Gehalt an 2,2'-MDI von 0,01 bis 20 Gew.-%, bezogen auf die Masse der Fraktion, abtrennt, und
d) gegebenenfalls aus der in Schritt c) erhaltenen Fraktion 4,4'-MDI zu 10 bis 98 % entfernt, und
e) aus der in Schritt c) oder in Schritt d) erhaltenen Fraktion 2,2'-MDI ganz oder teilweise abtrennt, wobei man eine Fraktion enthaltend 0 bis 0,4 Gew.-% 2,2'-MDI, 1 bis 95 Gew.-% 4,4'-MDI und 5 bis 98,6 Gew.-% 2,4'-MDI, bezogen auf die Masse der MDI-Isomeren, erhält, und optional in einer bevorzugten Ausführungsform
f) aus der Fraktion enthaltend 0 bis 0,4 Gew.-% 2,2'-MDI, 1 bis 95 Gew.-% 4,4'-MDI und 5 bis 98,6 Gew.-% 2,4'-MDI, bezogen auf die Masse der MDI-Isomeren, eine Fraktion enthaltend mindestens 99 Gew.-% 2-Kern-Methylendiphenyldiisocyanat bezogen auf die Masse der Fraktion und 0 bis 0,5 Gew.-% 2,2'-MDI, 0,1 bis 80 Gew.-% 4,4'-MDI und 20 bis 99,9 Gew.-% 2,4'-MDI, bezogen auf die Masse MDI-Isomeren, abtrennt.

Kern der Erfindung ist ein Verfahren zur gezielten Entfernung von 2,2'-MDI sowie Lösungsmittelresten und Phenylisocyanaten aus der monomeren Isomerenmischung durch ein Trennverfahren, insbesondere durch Destillation. Da Phenylisocyanate, Lösungsmittel wie Monochlorbenzol und ortho-Dichlorbenzol sowie 2,2'-MDI gegenüber 4,4'- und 2,4'-MDI einen niedrigeren Siedepunkt besitzen, reichem sie sich stets im Destillat einer Kolonne an. Prinzipiell sind jedoch auch Kristallisation oder Extraktion einsetzbar. Die auf diese Weise hergestellten bzw. gereinigten MDI-Gemische mit hohen Gehalten an 2,4'-MDI lassen sich gut in der Polyurethan-Herstellung einsetzen.

Das im erfindungsgemäßen Verfahren eingesetzte Polyamin bzw. Polyamingemisch der Diphenylmethanreihe wird in Schritt a) durch Kondensation von Anilin und Formaldehyd in Gegenwart eines sauren Katalysators erhalten (H. J. Twitchett, Chem. Soc. Rev. 3(2), 209 (1974); W. M. Moore in: Kirk-Othmer Encycl. Chem. Technol., 3^{rd}. Ed., New York, 2, 338-348 (1978)). Für das erfindungsgemäße Verfahren ist es dabei unerheblich, ob Anilin und Formaldehyd zunächst in Abwesenheit des sauren Katalysators vermischt werden und der saure Katalysator anschließend zugesetzt wird oder ob ein Gemisch aus Anilin und saurem Katalysator mit Formaldehyd zu Reaktion gebracht wird.

Geeignete Polyamingemische der Diphenylmethanreihe werden üblicherweise erhalten durch Kondensation von Anilin und Formaldehyd im Stoffmengenverhältnis 20 bis 1,6, bevorzugt 10 bis 1,8 sowie einem Stoffinengenverhältnis von Anilin und saurem Katalysator von 20 bis 1, bevorzugt 10 bis 2.

Formaldehyd wird technisch üblicherweise als wässrige Lösung eingesetzt. Hierbei kann der Wassergehalt von 1 bis 95 % variieren. Bevorzugt wird eine wässrige Lösung mit 50 bis 80 % Wasser eingesetzt. Es können jedoch auch andere methylengruppenliefernde Verbindungen wie z.B. Polyoxymethylenglykol, *para*-Formaldehyd oder Trioxan eingesetzt werden.

Als saure Katalysatoren haben sich starke organische und vorzugsweise anorganische Säuren bewährt. Geeignete Säuren sind beispielsweise Salzsäure, Schwefelsäure, Phosphorsäure, und Methansulfonsäure. Bevorzugt wird Salzsäure eingesetzt. Es können aber auch feste saure Katalysatoren wie beispielsweise organische und anorganische Ionenaustauscher, saure Silizium-Aluminium-Mischoxide sowie bevorzugt saure Zeolithe verwendet werden.

In einer bevorzugten Ausführungsform des Verfahrens wird Anilin und saurer Katalysator zunächst vermischt. Dieses Gemisch wird, gegebenenfalls nach Abfuhr von Wärme, in einem weiteren Schritt mit Formaldehyd bei Temperaturen zwischen 20°C und 100°C, bevorzugt bei 30°C bis 70°C in geeigneter Weise vermischt und anschließend in einem geeigneten Verweilzeitapparat einer Vorreaktion zu unterzogen. Die Vorreaktion erfolgt bei Temperaturen zwischen 20°C bis 100°C, vorzugsweise im Temperaturbereich 30°C bis 80°C. Im Anschluss an Vermischung und Vorreaktion wird die Temperatur des Reaktionsgemisches in Stufen oder kontinuierlich und gegebenenfalls unter Überdruck auf eine Temperatur von 100°C bis 250°C, bevorzugt auf 100°C bis 180°C, besonders bevorzugt auf eine Temperatur von 100°C bis 160°C gebracht.

Es ist jedoch ebenfalls möglich, in einer anderen Ausführungsform des Verfahrens Anilin und Formaldehyd zunächst in Abwesenheit des sauren Katalysators im Temperaturbereich von 5°C bis 130°C, bevorzugt von 40°C bis 100°C, besonders bevorzugt von 60°C bis 90°C, zu vermischen und zur Reaktion zu bringen. Hierbei bilden sich Kondensationsprodukte aus Anilin und Formaldehyd (sog. Aminal). Im Anschluss an die Aminalbildung kann im Reaktionsgemisch vorhandenes Wasser durch Phasentrennung oder andere geeignete Verfahrensschritte, beispielsweise durch Destillation, entfernt werden. Das Kondensationsprodukt wird dann in einem weiteren Verfahrensschritt mit dem sauren Katalysator in geeigneter Weise vermischt und in einem Verweilzeitapparat bei 20°C bis 100°C, bevorzugt 30°C bis 80°C einer Vorreaktion unterzogen. Anschließend wird die Temperatur des Reaktionsgemisches in Stufen oder kontinuierlich und gegebenenfalls unter Überdruck auf eine Temperatur von 100°C bis 250°C, bevorzugt auf 100°C bis 180°C, besonders bevorzugt auf eine Temperatur von 100°C bis 160°C gebracht.

Zur Aufarbeitung des sauren Reaktionsgemisches wird das Reaktionsgemisch nach dem Stand der Technik mit einer Base neutralisiert. Nach dem Stand der Technik erfolgt die Neutralisation üblicherweise bei Temperaturen von beispielsweise 90 - 100°C (H.J. Twitchett, Chem. Soc. Rev. 3(2), 223 (1974)). Als Basen sind beispielsweise geeignet die Hydroxide der Alkali- und Erdalkalielemente. Vorzugsweise kommt wässrige NaOH zur Anwendung.

Im Anschluss an die Neutralisation wird nach dem Stand der Technik die organische von der wässrigen Phase durch geeignete Verfahren (z.B. Phasentrennung in einer Scheideflasche) getrennt. Diese Trennung von organischer und wässriger Phase kann bei der selben Temperatur erfolgen, bei der die Neutralisation des sauren Umlagerungsgemisches erfolgte. Die nach Abtrennung der wässrigen Phase verbleibende produktenthaltende organische Phase wird nach dem Stand der Technik zur Abtrennung von Salzen und überschüssiger Base einer Wäsche unterzogen. Anschließend wird die gereinigte organische Phase von überschüssigem Anilin und anderen im Gemisch vorhandenen Stoffen (z.B. weiteren Lösungsmitteln) durch geeignete physikalische Trennverfahren wie z.B. Destillation, Extraktion oder Kristallisation befreit.

Das so erhaltene Polyamin der Diphenylmethanreihe (Roh-MDA) aus Schritt a) wird im Schritt b) nach den bekannten Methoden mit Phosgen in einem inerten organischen Lösungsmittel zu den entsprechenden Isocyanaten umgesetzt. Das Molverhältnis von Roh-MDA zu Phosgen wird zweckmäßigerweise so bemessen, dass pro Mol NH₂-Gruppe 1 bis 10 Mol, vorzugsweise 1,3 bis 4 Mol Phosgen in der Reaktionsmischung vorliegen. Als inerte Lösungsmittel haben sich chlorierte, aromatische Kohlenwasserstoffe wie z.B. Monochlorbenzol, Dichlorbenzole, Trichlorbenzole, die entsprechenden Toluole und Xylole sowie Chlorethylbenzol bewährt. Insbesondere Anwendung finden als inerte organische Lösungsmittel Monochlorbenzol, Dichlorbenzol oder Mischungen dieser Chlorbenzole. Die Menge an Lösungsmittel wird vorzugsweise so bemessen, dass die Reaktionsmischung einen Isocyanatgehalt von 2 bis 40 Gew.-%, vorzugsweise zwischen 5 uns 20 Gew.-%, bezogen auf das Gesamtgewicht der Reaktionsmischung, aufweist. Die Reaktion von Roh-MDA mit Phosgen wird hierbei bei Temperaturen von 50 bis 250°C und bei Drücken von Umgebungsdruck bis hin zu 50 bar durchgeführt. Bevorzugt wird bei 70 bis 180°C und 2 bis 20 bar gearbeitet.

Nach beendeter Phosgenierung werden aus der Reaktionsmischung das überschüssige Phosgen, das inerte organische Lösungsmittel, die gebildete HCl oder Mischungen davon durch geeignete Verfahren (z.B. Destillation) abgetrennt. Hierzu wird der Druck schrittweise bis ins Vakuum reduziert und der verbliebene Phosgenüberschuss und die gebildete HC1 verdampft und abgetrennt. Anschließend wird bevorzugt per Verdampfung das Lösungsmittel schrittweise reduziert unter weiterer Reduzierung des absoluten Drucks auf bis zu 1 mbar, bevorzugt auf bis zu 5 mbar. Parallel dazu wird die Temperatur erhöht bis das Lösungsmittel fast vollständig bis auf weit weniger als 0,1 % entfernt ist. Letztlich enthält man in diesem Schritt b) ein rohes Di- und Polyisocyanat (rohes MDI-Gemisch).

Aus dem rohen Di- und Polyisocyanat wird anschließend in der Polymer- / Monomer-Trennung in Schritt c) eine Fraktion enthaltend mindestens 95 Gew.-% 2-Kern-Methylendiphenyldiisocyanat mit einem Gehalt an 4,4'-MDI von 49 - 95,99 Gew.-%, einem Gehalt an 2,4'-MDI von 4 bis 45 Gew.-% und einem Gehalt an 2,2'-MDI von 0,01 bis 20 Gew.-%, bezogen auf die Masse der Fraktion, abgetrennt. Die Abtrennung dieser 2-Kern-MDI-Fraktion erfolgt dabei vorzugsweise ein- oder zweistufig. Hierbei wird bevorzugt thermisch bei 170 bis 230°C und absoluten Drücken von 0,1 bis 30 mbar die leichter siedende Fraktion mit hohen Gehalten an 2-Kern-MDI-Isomeren über Kopf verdampft und abgezogen. Bevorzugt betragen die Temperaturen 180 bis 220°C und die absoluten Drücke von 1 bis 15 mbar.

Bevorzugt enthält die in Schritt c) abgetrennte Fraktion 98 bis 100 Gew.-% 2-Kern-Methylendiphenyldiisocyanat mit einem Gehalt an 4,4'-MDI von 80 - 93,9 Gew.-%, einem Gehalt an 2,4'-MDI von 6 bis 19,9 Gew.-% und einem Gehalt an 2,2'-MDI von 0,1 bis 5 Gew.-%, bezogen auf die Masse der Fraktion. Besonders bevorzugt enthält die in Schritt c) abgetrennte Fraktion mindestens 99 Gew.-% 2-Kern-Methylendiphenyldiisocyanat mit einem Gehalt an 4,4'-MDI von 82 bis 92,8 Gew.-%, einem Gehalt an 2,4'-MDI von 7 bis 17,8 Gew.-% und einem Gehalt an 2,2'-MDI von 0,2 bis 3 Gew.-%, bezogen auf die Masse der Fraktion.

4,4'-MDI ist das in der Polyurethan-Herstellung am meisten eingesetzte MDI-Isomere. Aus diesem Grund kann es zweckmäßig sein, aus der in Schritt c) erhaltenen Fraktion das 4,4'-MDI zu einem großen Anteil zunächst abzutrennen. Bevorzugt wird daher in Schritt d) in einem Destillationsschritt oder einem Kristallisationsschritt 10 bis 98 Gew.-% des 4,4'-MDI, besonders bevorzugt 50 bis 95 Gew.-%, ganz besonders bevorzugt 75 bis 93 Gew.-% des 4,4'-MDI abgetrennt. Die in Schritt d) eingesetzte Destillationskolonne wird bevorzugt bei 170 bis 230°C und absoluten Drücken von 0,1 bis 30 mbar betrieben. Besonders bevorzugt wird der mit 4,4'-MDI angereicherte Strom im Sumpf einer Destillationskolonne abgezogen, wobei Temperaturen von 180 bis 220°C und absolute Drücke von 1 bis 15 mbar eingestellt werden. Schritt d) kann alternativ mittels einer oder mehrerer Kristallisationsschritte bei 30 bis 40°C durchgeführt werden, wobei die 4,4'-reiche Fraktion als Kristallisat und die 2,4'- und 2,2'-reiche Fraktion d) als Mutterlauge anfällt. In diesem Fall werden bevorzugt ebenfalls 10 bis 98 Gew.-%, besonders bevorzugt 50 bis 95 Gew.-%, ganz besonders bevorzugt 75 bis 93 Gew.-% des zugeführten 4,4'-MDI in der Kristallisation als Kristallisat abgetrennt.

In Schritt e) wird dann aus der in Schritt c) erhaltenen Fraktion enthaltend mindestens 95 Gew.-% 2-Kern-Methylendiphenyldiisocyanat mit einem Gehalt an 4,4'-MDI von 49 bis 95,99 Gew.-%, einem Gehalt an 2,4'-MDI von 4 bis 45 Gew.-% und einem Gehalt an 2,2'-MDI von 0,01 bis 20 Gew.-%, bezogen auf die Masse der Fraktion, gegebenenfalls nach vorheriger Abtrennung von großen Teilen des 4,4'-MDI in Schritt d), das 2,2'-MDI ganz oder teilweise abgetrennt.

Die im Schritt e) als Destillat gewonnene 2,2'-reiche Fraktion enthält mindestens 99 Gew.-% 2-Kern-MDI und 10 bis 98 Gew.-% 2,2'-MDI, 2 bis 90 Gew.-% 2,4'-MDI sowie 0 bis 30 Gew.-% 4,4'-MDI bezogen auf die Masse der MDI-Isomeren. Bevorzugt enthält die 2,2'-MDI-reiche Fraktion 20 bis 95 Gew.-% 2,2'-MDI, 5 bis 80 Gew.-% 2,4'-MDI sowie 0 bis 20 Gew.-% 4,4'-MDI, besonders bevorzugt von 50 bis 85 Gew.-% 2,2'-MDI, 15 bis 50 Gew.-% 2,4'-MDI sowie 0 bis 10 Gew.-% 4,4'-MDI bezogen auf die Masse der MDI-Isomeren. Hierdurch werden aus der mit dem rohen Di- und Polyisocyanat in die Destillation in Schritt c) insgesamt eingebrachten Menge an 2,2'-MDI bevorzugt 50 bis 99,9999 % (100 %), besonders bevorzugt 65 bis 99,99 %, ganz besonders bevorzugt 80 bis 99,9 % in Schritt e) als Destillatstrom entnommen.

Die in Schritt e) abgetrennte 2,2'-MDI-Fraktion wird dabei in einem weiteren Schritt per Isomerendestillation in eine Leichtsiederfraktion sowie eine 2,2'-MDI reiche Fraktion zerlegt. Über Kopf als Destillat werden die störenden Leichtsieder wie Derivate von Phenylisocyanat, Lösungsmittelbestandteile und sonstige leichtflüchtige Nebenprodukte aus der MDA- und MDI-Herstellung wie beispielsweise Acridin entfernt. Hierzu wird eine Teilmenge des MDIs, beispielsweise 5 bis 30 % des Zulaufes, bevorzugt 10 bis 20 % des Zulaufes, als Schleppmittel im Destillatstrom eingesetzt. Die resultierende höhersiedende Fraktion enthält den größten Teil der MDI-Wertprodukte. Analog können die störenden Leichtsieder als Mutterlauge in einem oder mehreren Kristallationsschritten vom MDI-Wertprodukt abgetrennt werden.

Dabei wird ein MDI-Isomerengemisch mit hohen Gehalten an 2,2'-MDI von 5 bis 99,99 Gew.-%, bevorzugt 10 bis 95 Gew.-%, besonders bevorzugt 20 bis 90 Gew.-%, ganz besonders bevorzugt 30 bis 85 Gew.-% bezogen auf die Masse der MDI-Isomeren durch destillative Abtrennung von störenden Leichtsiedern erhalten. Diese Isomerengemische mit hohen Gehalten an 2,2'-MDI von 5 bis 99,99 Gew.-% 2,2'-MDI, 0 bis 50 Gew.-% 4,4'-MDI und 0,01 bis 95 Gew.-% 2,4'-MDI, bevorzugt 10 bis 95 Gew.-% 2,2'-MDI, 0,1 bis 89,99 Gew.-% 2,4'-MDI und 0,01 bis 50 Gew.-% 4,4'-MDI bezogen auf die Masse der MDI-Isomere können dann mit Polyethern oder Polyestern zu Polyurethanen oder Prepolymeren umgesetzt werden. Alternativ können diese Isomerengemische mit hohen Gehalten an 2,2'-MDI auch zu MDI-Mischprodukten zur Aufstockung des 2,2'- und 2,4'-Gehaltes eingesetzt werden sowie zu deren Verarbeitung mit Polyethern oder Polyestern zu Polyurethanen oder Prepolymeren.

Die Abtrennung des 2,2'-MDI erfolgt im Schritt e) dabei bevorzugt in einer Destillationskolonne mit mindestens 10, besonders bevorzugt mindestens 15, ganz besonders bevorzugt mindestens 20 theoretischen Stufen im Abtriebsteil der Kolonne zur Minimierung des Gehalts an 2,2'-MDI im Sumpf. Hierzu werden Rücklaufverhältnisse (Destillatrücklauf in die Kolonne / entnommene Destillatmenge) am Kopf der Kolonne von 0,5 bis 500 eingestellt. Bevorzugt werden Rücklaufverhältnisse von 2 bis 100 eingestellt. Der absolute Betriebsdruck einer solchen Kolonne wird bei 0,5 bis 30 mbar, bevorzugt bei 1 bis 15 mbar eingestellt.

Alternativ kann auch eine Kolonne eingesetzt werden, in der die von 2,2'-MDI abgereicherte Fraktion im Seitenstrom entnommen wird. In diesem Fall muss der Abtriebsteil der Kolonne zwischen der Zulaufstelle des Stromes aus Schritt c) oder Schritt d) und Entnahmestelle des abgereicherten 2,2'-MDI-Produktes eine Trennleistung von mindestens 10, bevorzugt mindestens 15, besonders bevorzugt mindestens 20 theoretischen Stufen aufweisen. Die Entnahme der 2,2'-MDIarmen Fraktion aus dem Sumpf kann dabei gasförmig oder flüssig erfolgen. Hierzu werden Rücklaufverhältnisse (Destillatrücklauf in die Kolonne / entnommene Destillatmenge) am Kopf der Kolonne von 0,5 bis 500 eingestellt. Bevorzugt werden Rücklaufverhältnisse von 2 bis 100 verwendet. Der absolute Betriebsdruck einer solchen Kolonne wird bei 0,5 bis 30 mbar, bevorzugt bei 1 bis 15 mbar eingestellt.

Nach Abtrennung des 2,2'-MDI wird in Schritt e) eine Fraktion enthaltend 0 bis 0,4 Gew.-% 2,2'-MDI, 1 bis 95 Gew.-% 4,4'-MDI und 5 bis 98,6 Gew.-% 2,4'-MDI, bezogen auf die MDI-Isomeren, erhalten.

Bevorzugt enthält die in Schritt e) erhaltene Fraktion 0 bis 0,3 Gew.-% 2,2'-MDI, 5 bis 85 Gew.-% 4,4'-MDI und 15 bis 95 Gew.-% 2,4'-MDI, bezogen auf die Masse der MDI-Isomeren, besonders bevorzugt 0 bis 0,18 Gew.-% 2,2'-MDI, 7 bis 75 Gew.-% 4,4'-MDI und 25 bis 93 Gew.-% 2,4'-MDI bezogen auf die Masse der MDI-Isomeren. Ganz besonders bevorzugt enthält die gewonnene Fraktion 0 bis 0,10 Gew.-% 2,2'-MDI, 30 bis 70 Gew.-% 4,4'-MDI und 30 bis 70 Gew.-% 2,4'-MDI bezogen auf die Masse der MDI-Isomeren. Gegebenenfalls kann bereits in diesem Schritt, beispeilsweise mittels einer Partialkondensation des Dampfes oberhalb der Sumpfphase der Kolonne, die gewünschte 2,4'-reiche und 2,2'-arme Fraktion als Endprodukt entnommen und die separate Fraktionierung in Schritt f) eingespart werden. Dies kann eine bevorzugte Ausführungsform zur Herstellung von 2,2'-armem eutektischem Gemisch 2,4'-/4,4'-MDI sein mit 0 bis 0,40 Gew.-% 2,2'-MDI, 30 bis 70 Gew.-% 4,4'-MDI und 30 bis 70 Gew.-% 2,4'-MDI bezogen auf die Masse der MDI-Isomeren, besonders bevorzugt mit 0 bis 0,20 Gew.-% 2,2'-MDI, 35 bis 60 Gew.-% 4,4'-MDI und 40 bis 65 Gew.-% 2,4'-MDI.

Optional wird in einer bevorzugten Ausführungsform in Schritt f) aus der in Schritt e) erhaltenen Fraktion enthaltend 0 bis 0,4 Gew.-% 2,2'-MDI, 1 bis 95 Gew.-% 4,4'-MDI und 5 bis 98,6 Gew.-% 2,4'-MDI, bezogen auf die MDI-Isomeren, schließlich durch Destillation eine Fraktion enthaltend mindestens 99 Gew.-% 2-Kern-MDI bezogen auf die Masse der Fraktion und 0 bis 0,5 Gew.-% 2,2'-MDI, 0,1 bis 80 Gew.-% 4,4'-MDI und 20 bis 99,9 Gew.-% 2,4'-MDI, bezogen auf die Masse der MDI-Isomeren, abtrennt. Bevorzugt wird die Destillation bei 0,5 bis 30 mbar, bevorzugt bei 1 bis 15 mbar absolutem Druck betrieben. Für die Destillation wird eine Kolonne mit mindestens 1, bevorzugt mindestens 5 und besonders bevorzugt mindestens 10 theoretischen Stufen eingesetzt. Alternativ kann die Fraktion aus Schritt e), wenn sie einen Gehalt an 2,4'-MDI von mehr als 60 Gew.-% bezogen auf die Massen der MDI-Isomeren aufweist, auch durch Kristallisation aufgereinigt werden. Hierbei fällt bei partieller Kristallisation die gewünschte Kristallphase mit hohen Gehalten an 2,4'-MDI und eine Mutterlauge mit hohen Gehalten an 4,4'-MDI an.

In Schritt f) können verschiedene 2-Kern-MDI-Mischungen mit verschiedenen Gehalten an 2,4'-MDI und 4,4'-MDI destillativ und mit sehr geringem Gehalt an 2,2'-MDI hergestellt werden. Je nach Anforderung kann beispielsweise in einer Destillationskolonne kampagnenweise ein mittel- oder hochkonzentriertes 2,4'-MDI-Produkt hergestellt werden. Alternativ dazu können auch zwei oder mehr Destillationskolonnen nebeneinander betrieben werden, die eine simultane Produktion der verschiedenen 2-Kern-MDI-Mischungen erlauben.

Bevorzugt enthalten die in Schritt f) erhaltenen Fraktionen 0 bis 0,35 Gew.-% 2,2'-MDI, 0,2 bis 60 Gew.-% 4,4'-MDI und 40 bis 99,8 Gew.-% 2,4'-MDI, bezogen auf die Masse der MDI-Isomeren. Besonders bevorzugt wird eine Fraktion enthaltend 0 bis 0,2 Gew.-% 2,2'-MDI, 0,5 bis 55 Gew.-% 4,4'-MDI und 45 bis 99,5 Gew.-% 2,4'-MDI, bezogen auf die Masse der MDI-Isomeren, erhalten. Ganz besonders bevorzugt wird in Schritt f) eine Fraktion enthaltend 0 bis 0,2 Gew.-% 2,2'-MDI, 0,5 bis 10 Gew.-% 4,4'-MDI und 90 bis 99,5 Gew.-% 2,4'-MDI, bezogen auf die Masse der MDI-Isomere, oder eine Fraktion enthaltend 0 bis 0,2 Gew.-% 2,2'-MDI, 35 bis 60 Gew.-% 4,4'-MDI und 40 bis 65 Gew.-% 2,4'-MDI, bezogen auf die Masse der MDI-Isomere, erhalten. Hierbei wird eine Reinheit der MDI-Isomere in der Fraktion von > 99,9 Gew.-% erreicht.

Der Gehalt an Lösungsmittel in der in Schritt f) erhaltenen Fraktion liegt dabei vorzugsweise zwischen 0 und 5 ppm, besonders bevorzugt zwischen 0 und 1 ppm, ganz besonders bevorzugt zwischen 0 und 0,5 ppm, bezogen auf die Masse der MDI-Isomeren. Der Gehalt an Phenylisocyanat in der in Schritt f) erhaltenen Fraktion liegt dabei vorzugsweise zwischen 0 und 5 ppm, besonders bevorzugt zwischen 0 und 1 ppm, ganz besonders bevorzugt zwischen 0 und 0,5 ppm, bezogen auf die Masse der MDI-Isomeren.

Die Erfindung betrifft auch ein Verfahren zur Herstellung von hochreinem 2,4'-MDI, bei dem die Fraktion e) oder die Fraktion f) mit 30 bis 70 Gew.-% 4,4'-MDI in einer weiteren Isomerendestillation zu einem hochreinen 2,4'-MDI mit 0 bis 10 Gew.-% 4,4'-MDI, bevorzugt 0 bis 5 Gew.-% 4,4'-MDI, besonders bevorzugt 0 bis 3 Gew.-% 4,4'-MDI bezogen auf das Gemisch aufkonzentriert wird und 0 bis 1, Gew.-% 2,2'-MDI, bevorzugt 0 bis 0,5 Gew.-% 2,2'-MDI und besonders bevorzugt 0 bis 0,2 Gew.-% 2,2'-MDI bezogen auf das Gemisch enthält.

Die Erfindung betrifft auch ein Verfahren, in dem beispielsweise die Schritte d) und e) oder die Schritte e) und f) und/oder die Schritte f) und die Abtrennung der Leichtsieder aus der in Schritt e) abgetrennten 2,2'-MDI-Fraktion in einer Seitenstromkolonne mit drei Entnahmeströmen durchgeführt werden und letztlich identische Endproduktzusammensetzungen enthalten werden. In diesem Fall würden lediglich einige der Zwischenprodukte nicht explizit separiert.

Die Erfindung betrifft auch ein Verfahren zur Herstellung von Prepolymeren oder Polyurethanen, bei denen die in Schritt f) hergestellte Fraktion enthaltend 0 bis 0,5 Gew.-% 2,2'-MDI, 0,1 bis 80 Gew.-% 4,4'-MDI und 20 bis 99,9 Gew.-% 2,4'-MDI, bezogen auf die MDI-Isomeren, mit Polyethern oder Polyestern umgesetzt wird.

Die Erfindung betrifft auch ein Verfahren zur Herstellung von Prepolymeren oder Polyurethanen, bei dem die in Schritt e) hergestellte Fraktion oder das hochreine 2,4'-MDI mit 0 bis 10 Gew.-% 4,4'-MDI und 0 bis 1 Gew.-% 2,2'-MDI mit Polyethern oder Polyestern umgesetzt wird.

### Beispiele:

Das erfindungsgemäße Verfahren wird nachfolgend anhand der Beispiele erläutert. Dabei sind alle %-Angaben auf Gewicht bezogen (Gew.-%). Für die Herstellung von 2,2'- und 2,4'-MDI-haltigem Isomerengemischen wird zunächst konventionell eine MDA-Base (Di- und Polyamine der Diphenylmethanreihe) aus Anilin und Formaldehydlösung hergestellt. Die MDA-Base wird anschließend phosgeniert und destillativ in eine Monomer- und Polymerfraktion zerlegt. Die so erhaltene Monomerfraktion (2-Kern-MDI) wird destillativ in die Isomeren zerlegt. Die angegebenen Analysenergebnisse der MDI-Isomere sowie der Nebenprodukte Monochlorbenzol und Phenylisocyanat wurden gaschromatographisch gemessen. Der HC-Gehalt (Gehalt an hydrolysierbarem Chlor) wurde titrimetrisch ermittelt.

### Beispiel 1 (nicht erfindungsgemäß):

Im Schritt a) werden 1000 g Anilin mit 306 g 31,9 %iger, wässriger HCl in einem Rührkessel bei 40°C vermischt. Hierzu wird in 15 Minuten 480 g 32 %ige Formaldehyd-Lösung zugetropft. Es wird zunächst weitere 15 min bei 40°C gerührt und die Temperatur langsam auf 100°C innerhalb der nächsten 2,5 h erhöht. Anschließend wird über 10 h bei 100°C am Rückfluss gerührt und die Reaktionsmischung mit 50 %iger Natronlauge neutralisiert, die wässrige Phase abgetrennt und die organische Phase mit Wasser nachgewaschen. Die organische Lösung wird entnommen und im Vakuum destillativ von überschüssigem Anilin befreit.

Im Schritt b) wird das MDA-Reaktionsprodukt in einem 2. Rührkessel in die vorgelegte eiskalte 15 %ige Lösung von Phosgen in Monochlorbenzol (MCB) gegossen; der molare Überschuss an Phosgen beträgt 200 %. Die Reaktionslösung wird unter kontinuierlicher Zudosierung von 40 Liter/h Phosgen langsam innerhalb einer Stunde auf 100°C aufgeheizt. Innerhalb einer weiteren Stunde wird das Gemisch auf Siedetemperatur gebracht, die Phosgendosierung gestoppt und Vakuum angelegt. Schrittweise wird die Temperatur auf 210°C erhöht, der Druck auf 3 mbar absolut abgesenkt und das Lösungsmittel vollständig entfernt. Man erhält ein rohes MDI-Gemisch mit 58 Gew.-% monomerem MDI; bezogen auf die Masse an MDI-Isomeren und -Oligomeren.

Die rohe MDI-Fraktion b) wird anschließend in Schritt c) in das polymere MDI-Produkt sowie die Monomer-Fraktion (2-Kern-MDI) zerlegt. Als Versuchsaufbau dient ein Glaskolben mit einer Tropfenabscheiderpackung im Dampfraum. Über Kopf wird das Destillat ausgetrieben, vollständig niedergeschlagen und entnommen. Der Druck wird auf 5 mbar absolut eingestellt. Der Zulauf von rohem MDI-Gemisch wird mit 180°C der kontinuierlich betriebenen Apparatur zugeführt. Im stationären Gleichgewicht werden die folgenden Zusammensetzungen der Produkte gemessen:
Sumpf: polymeres MDI-Gemisch mit einer Viskosität von 185 mPas bei 25°C
Kopfstrom der Destillationsapparatur [=Fraktion c)]:
0,54 % 2,2'-MDI, 11,29 % 2,4'-MDI, 88,17 % 4,4'-MDI, bezogen auf das Gewicht aller MDI-Isomere.

Die Fraktion c) stellt das Ausgangsgemisch für die folgende Isomerentrennung dar.

Die Fraktion c) wird in Schritt d) nun einer kontinuierlich arbeitenden Labor-Packungskolonne (Isomerendestillation) mit je 10 theoretischen Trennstufen in Ab- und Auftriebsteil zugeführt. Der Druck wird bei 3 mbar am Kopf der Kolonne eingestellt. Die Zulauftemperatur beträgt 175°C. Das über Kopf abdestillierte Produkt wird von einem Kondensator bei 100°C kondensiert. Das kondensierte MDI wird partiell über einen Verteiler zurück in die Destillation gegeben und der Rest als Destillatfraktion entnommen. Bei einem Rücklaufverhältnis (Rücklauf/Destillat) von 7 und einer Abnahme von 20 % des Feeds als Destillat und 80 % als Sumpf erhält man eine Sumpfzusammensetzung mit 0 % 2,2'-MDI, 1,4 % 2,4'-MDI, 98,6 % 4,4'-MDI. Die Fraktion d) erreicht eine Destillatqualität von 2,2 % 2,2'-MDI, 41,9 % 2,4'-MDI, 55,9 % 4,4'-MDI, beide bezogen auf die jeweilige Masse der MDI-Isomere.

Diese Destillatfraktion d) wird nun einer weiteren Glaskolonne zur Destillation zugeführt. Diese Glaskolonne besitzt je 10 theoretische Trennstufen in Abtriebs- und Verstärkerteil. Die Fraktion d) wird in diesem Schritt e) nun zwischen Abtriebs- und Verstärkerteil bei 175°C kontinuierlich zudosiert. Bei ebenfalls 3 mbar absolut wird nun über Kopf 6 % des Zulaufes bei einem Rücklaufverhältnis von 12 abgezogen. Es entsteht eine Destillatphase mit 18,4 % 2,2'-MDI, 70,6 % 2,4'-MDI, 11,0 % 4,4'-MDI bezogen auf die Masse der MDI-Isomere. Weiterhin enthält die Destillatphase 0,3 % organische Verbindungen, die aus Nebenprodukten bei der MDI-Herstellung stammen. Die Sumpffraktion e) besitzt die Zusammensetzung 1,2 % 2,2'-MDI, 40,1 % 2,4'-MDI, 58,7 % 4,4'-MDI, bezogen auf die Masse der MDI-Isomere.

Die Fraktion e) wird nun im abschließenden Schritt f) destillativ feingereinigt und in einer weiteren Glaskolonne mit je 10 theoretische Trennstufen in Abtriebs- und Verstärkerteil zerlegt. Die Fraktion e) wird zwischen Abtriebs- und Verstärkerteil bei 175°C kontinuierlich zudosiert. Bei 3 mbar absolut wird über Kopf 2/3 des Zulaufes bei einem Rücklauf-verhältnis von 1 abgezogen. Es entsteht eine Destillatphase mit 1,79 % 2,2'-MDI, 52,74 % 2,4'-MDI, 45,47 % 4,4'-MDI bezogen auf die Masse der MDI-Isomere sowie 1 ppm Monochlorbenzol, 1 ppm Phenylisocyanat und 10 ppm hydrolysierbares Chlor. Die resultierende Sumpfphase wird mit dem rohen MDI-Gemisch, der Fraktion b) in Beispiel 1, vereinigt und wieder den Schritten c) bis f) zugeführt.

### Beispiel 2 (erfindungsgemäß):

Die Destillatfraktion d) aus dem Beispiel 1 wird im Schritt e) einer weiteren Isomerentrennung per Destillation zugeführt. Diese Glaskolonne besitzt 10 theoretische Trennstufen im Verstärkerteil und 20 theoretische Stufen im Abtriebsteil (Packung unterhalb des Zulaufverteilers). Die Fraktion d) wird zwischen Abtriebs- und Verstärkerteil bei 175°C kontinuierlich zudosiert. Bei ebenfalls 3 mbar absolut wird nun über Kopf 6 % des Zulaufes bei einem Rücklaufverhältnis von 10 abgezogen. Es entsteht eine Destillatphase mit 36,8 % 2,2'-MDI, 62,4 % 2,4'-MDI, 0,8 % 4,4'-MDI bezogen auf die Masse der MDI-Isomere. Weiterhin enthält die Destillatphase 0,3 % organische Verbindungen, die aus Nebenprodukten bei der MDI-Herstellung stammen. Die Sumpffraktion e) besitzt die Zusammensetzung von 0,05 % 2,2'-MDI, 40,65 % 2,4'-MDI, 59,3 % 4,4'-MDI, bezogen auf die Masse der MDI-Isomere.

Die Fraktion e) wird nun im abschließenden Schritt f) destillativ feingereinigt und in einer weiteren Glaskolonne mit je 10 theoretische Trennstufen in Abtriebs- und Verstärkerteil zerlegt. Die Fraktion e) wird zwischen Abtriebs- und Verstärkerteil bei 175°C kontinuierlich zudosiert. Bei 3 mbar absolut wird über Kopf 2/3 des Zulaufes bei einem Rücklaufverhältnis von 1 (Destillatstrom = Rücklaufstrom) abgezogen. Es entsteht eine Destillatphase, die Fraktion f) als Zielprodukt, mit 0,07 % 2,2'-MDI, 52,52 % 2,4'-MDI, 47,41 % 4,4'-MDI bezogen auf die Masse der MDI-Isomere sowie 0,2 ppm Monochlorbenzol, 0,1 ppm Phenylisocyanat und 7 ppm hydrolysierbares Chlor. Die resultierende Sumpfphase wird mit dem rohen MDI-Gemisch, der Fraktion b) in Beispiel 1, vereinigt und wieder den Schritten c) bis f) zugeführt.

### Beispiel 3 (nicht erfindungsgemäß):

Die Fraktion e) aus dem Beispiel 1 wird im Schritt f) destillativ feingereinigt und in einer weiteren Glaskolonne mit je 10 theoretischen Trennstufen in Abtriebs- und Verstärkerteil zerlegt. Die Fraktion e) wird zwischen Abtriebs- und Verstärkerteil bei 175°C kontinuierlich zudosiert. Bei 3 mbar absolut wird über Kopf 35 % des Zulaufes bei einem Rücklaufverhältnis von 2 (Destillatstrom = Rücklaufstrom) abgezogen. Es entsteht eine Destillatphase, die Fraktion f) als Zielprodukt, mit 3,37 % 2,2'-MDI, 95,12 % 2,4'-MDI, 1,51 % 4,4'-MDI bezogen auf die Masse der MDI-Isomere. Die resultierende Sumpfphase wird mit dem rohen MDI-Gemisch, der Fraktion b) in Beispiel 1, vereinigt und wieder den Schritten c) bis f) zugeführt.

### Beispiel 4 (erfindungsgemäß):

Die Fraktion e) aus dem Beispiel 2 wird nun im abschließenden Schritt f) destillativ feingereinigt. Hierzu wird mit einer weiteren Glaskolonne mit je 10 theoretischen Trennstufen in Abtriebs- und Verstärkerteil gearbeitet. Die Fraktion e) wird zwischen Abtriebs- und Verstärkerteil bei 175°C kontinuierlich zudosiert. Bei 3 mbar absolut wird über Kopf 30 % des Zulaufes bei einem Rücklaufverhältnis von 2 abgezogen. Es entsteht eine Destillatphase, die Fraktion f) als Zielprodukt, mit 0,16 % 2,2'-MDI, 97,05 % 2,4'-MDI, 2,79 % 4,4'-MDI bezogen auf die Masse der MDI-Isomere. Die resultierende Sumpfphase wird mit dem rohen MDI-Gemisch, der Fraktion b) in Beispiel 1, vereinigt und wieder den Schritten c) bis f) zugeführt.

**Tabelle 1:**

| Analysedaten der hergestellten Fraktionen aus Verfahrensschritt f) der Beispiele 1-4 in Gew.-% lt. GC-Analyse. | | | | |
|---|---|---|---|---|
| **Beispiel-Nr.** | **1** | **2** | **3** | **4** |
| 2,2'-MDI | 1,79 | 0,07 | 3,37 | 0,16 |
| 2,4'-MDI | 52,74 | 52,52 | 95,12 | 97,05 |
| 4,4'-MDI | 45,47 | 47,41 | 1,51 | 2,79 |

### Beispiel 5 (erfindungsgemäß):

Das Endprodukt des Beispiels 2, die Destillatfraktion f) wird in einem weiteren Schritt destillativ zu einer hochreinen Fraktion 2,4'-MDI aufkonzentriert. Hierzu wird erneut eine Glaskolonne mit je 10 theoretischen Trennstufen in Abtriebs- und Verstärkerteil gearbeitet. Die Fraktion f) aus Beispiel 2 wird zwischen Abtriebs- und Verstärkerteil bei 175°C kontinuierlich zudosiert. Bei 3 mbar absolut wird über Kopf 50 % des Zulaufes bei einem Rücklaufverhältnis von 2 abgezogen. Es entsteht eine Destillatphase, die Fraktion f) als Zielprodukt, mit 0,15 % 2,2'-MDI, 96,86 % 2,4'-MDI, 2,99 % 4,4'-MDI bezogen auf die Masse der MDI-Isomere. Die resultierende Sumpfphase wird mit dem rohen MDI-Gemisch, der Fraktion b) in Beispiel 1, vereinigt und wieder den Schritten c) bis f) zugeführt.

### Beispiel 6 (erfindungsgemäß):

Die Destillatfraktion e) des Beispiels 2 wird in einem weiteren Schritt destillativ gereinigt zu einer verwertbaren MDI-Fraktion g). Hierzu wird erneut eine Glaskolonne mit je 10 theoretischen Trennstufen in Abtriebs- und Verstärkerteil gearbeitet. Die Destillatfraktion aus Schritt e) des Beispiels 2 wird zwischen Abtriebs- und Verstärkerteil bei 175°C kontinuierlich zudosiert. Bei 3 mbar absolut wird über Kopf 20 % des Zulaufes bei einem Rücklaufverhältnis von 5 abgezogen. Es entsteht eine Sumpfphase, die Fraktion g) als Zielprodukt, mit 28,2 % 2,2'-MDI, 70,9 % 2,4'-MDI, 0,9 % 4,4'-MDI bezogen auf die Masse der MDI-Isomere. Die resultierende Destillatphase enthält neben dem Hauptbestandteil 2,2'-MDI sehr viele leichtsiedende Nebenkomponenten der MDI-Produktion und wird der thermischen Verwertung zugeführt.

Obige Beispiele zeigen, dass durch Einsatz eines extrem vielstufigen Abtriebsteils in der Trennkolonne des Verfahrensschritts e) die 2,2'-MDI-Konzentration im MDI-Isomerengemisch minimiert werden kann. Das Produkt einer so verbesserten Isomerentrennkolonne kann zur Herstellung von weitgehend 2,2'-freien MDI-Isomerenmischungen benutzt werden, beispielsweise für ein hochreines 2,4'-Produkt bzw. für ein Gemisch aus 2,4'- und 4,4'-Isomer in der Nähe des eutektischen Mischpunktes. Weiterhin wird gezeigt, dass das abgetrennte 2,2'-reiche MDI-Gemisch durch nochmalige Destillation von den störenden Leichtsiedern befreit und für die PolyurethanChemie verfügbar gemacht und so seine Verbrennung minimiert und die MDI-Ausbeute gesteigert werden kann.

Das folgende Beispiel 7 beschreibt exemplarisch für eine Anwendung die verbesserten Eigenschaften von Polyurethansystemen auf der Basis hoch 2,4'-MDI-haltigen MDI-Produkten mit minimiertem 2,2'-MDI-Gehalt.

### Beispiel 7:

Anwendungsbeispiel für die in den Beispielen 1-4 hergestellten erfindungsgemäßen (mit minimiertem 2,2'-MDI-Gehalt) und nicht erfindungsgemäßen hoch 2,4'-MDI-haltigen MDI-Produkte. Es wird ein Klebstoff für zwei Folien hergestellt und die Emission von Restmonomeren aus dem Verbund als Maß für die Reaktivität dargestellt. Bevorzugt wird von Folienverbundherstellern, dass die Emissionen insgesamt so niedrig wie möglich und die Lagerzeit bis zum vollständigen Reaktionsabschluss so kurz wie möglich ist.

### Herstellung von Prepolymer I

515 g Desmophen 1112 BD ® (Bayer AG) mit einer Hydroxylzahl von 112 mg KOH/g und einem Wassergehalt von 0,03 Gew.-% werden unter Stickstoff bei 60°C zu 486 g eines nach Beispiel 1 hergestellten Diphenymethandiisocyanats mit folgender Zusammensetzung gegeben:

| |
|---|
| 52,74 Gew.-% 2,4'-Diphenylmethandiisocyanat |
| 1,79 Gew.-% 2,2'- Diphenylmethandiisocyanat |
| 45,47 Gew.-% 4,4'- Diphenylmethandiisocyanat |

Nach Abklingen der leicht exothermen Reaktion wird die Reaktion bei 75°C zu Ende geführt. Nach 7 h erhält man einen konstanten Isocyanatgehalt. Analysendaten:

| | |
|---|---|
| NCO-Gehalt (Gew.-%) | 11,68 |
| Viskosität bei 25°C (mPas) | 5210 |
| Gehalt an 2,2'-MDI (Gew.-%) | 0,8 |
| Gehalt an 2,4'-MDI (Gew.-%) | 14,9 |
| Gehalt an 4,4'-MDI (Gew.-%) | 8,7 |

### Herstellung von Prepolymer II

515 g Desmophen 1112 BD ® (Bayer AG) mit einer Hydroxylzahl von 112 mg KOH/g und einem Wassergehalt von 0,03 Gew.-% werden unter Stickstoff bei 60°C zu 486 g eines nach dem erfmdungsgemäßen Verfahren nach Beispiel 2 hergestellten Diphenymethandiisocyanats mit folgender Zusammensetzung gegeben:

| | |
|---|---|
| 52,52 Gew.-% | 2,4'-Diphenylmethandiisocyanat |
| 0,07 Gew.-% | 2,2'- Diphenylmethandiisocyanat |
| 47,41 Gew.-% | 4,4'- Diphenylmethandiisocyanat |

Nach Abklingen der leicht exothermen Reaktion wird die Reaktion bei 75°C zu Ende geführt. Nach 7 h erhält man einen konstanten Isocyanatgehalt. Analysendaten:

| | |
|---|---|
| NCO-Gehalt Gew.-%) | 11,71 |
| Viskosität bei 25°C (mPas) | 4180 |
| Gehalt an 2,2'-MDI (Gew.-%) | <0,05 |
| Gehalt an 2,4'-MDI (Gew.-%) | 15,1 |
| Gehalt an 4,4'-MDI (Gew.-%) | 9,5 |

### Herstellung von Prepolymer III

515 g Desmophen 1112 BD ® (Bayer AG) mit einer Hydroxylzahl von 112 mg KOH/g und einem Wassergehalt von 0,03 Gew.-% werden unter Stickstoff bei 60°C zu 486 g eines nach Beispiel 3 hergestellten Diphenymethandiisocyanats mit folgender Zusammensetzung gegeben:

| | |
|---|---|
| 95,12 Gew.-% | 2,4'-Diphenylmethandiisocyanat |
| 3,37 Gew.-% | 2,2'- Diphenylmethandiisocyanat |
| 1,51 Gew.-% | 4,4'- Diphenylmethandiisocyanat |

Nach Abklingen der leicht exothermen Reaktion wird die Reaktion bei 75°C zu Ende geführt. Nach 7 h erhält man einen konstanten Isocyanatgehalt. Analysendaten:

| | |
|---|---|
| NCO-Gehalt Gew.-%) | 11,81 |
| Viskosität bei 25°C (mPas) | 4310 |
| Gehalt an 2,2'-MDI (Gew.-%) | 1,2 |
| Gehalt an 2,4'-MDI (Gew.-%) | 22,7 |
| Gehalt an 4,4'-MDI (Gew.-%) | <0,05 |

### Herstellung von Prepolymer IV

515 g Desmophen 1112 BD ® (Bayer AG) mit einer Hydroxylzahl von 112 mg KOH/g und einem Wassergehalt von 0,03 Gew.-% werden unter Stickstoff bei 60°C zu 486 g eines nach dem erfindungsgemäßen Verfahren nach Beispiel 4 hergestellten Diphenymethandiisocyanats mit folgender Zusammensetzung gegeben:

| | |
|---|---|
| 97,05 Gew.-% | 2,4'-Diphenylmethandiisocyanat |
| 0,16 Gew.-% | 2,2'- Diphenylmethandiisocyanat |
| 2,79 Gew.-% | 4,4'- Diphenylmethandiisocyanat |

Nach Abklingen der leicht exothermen Reaktion wird die Reaktion bei 75°C zu Ende geführt. Nach 7 h erhält man einen konstanten Isocyanatgehalt. Analysendaten:

| | |
|---|---|
| NCO-Gehalt Gew.-%) | 11,74 |
| Viskosität bei 25°C (mPas) | 4440 |
| Gehalt an 2,2'-MDI (Gew.-%) | <0,05 |
| Gehalt an 2,4'-MDI (Gew.-%) | 23,0 |
| Gehalt an 4,4'-MDI (Gew.-%) | 0,4 |

### Herstellung einer Polyolmischung

1000 g Baycoll AD 1115 ® (Bayer AG) mit einer Hydroxylzahl von 113,3 mg KOH/g, Säurezahl 0,8 mg KOH/g und einem Wassergehalt von 0,03 Gew.-% und 80 g Trimethylolpropan werden intensiv gemischt. Die dabei entstehende Polyolmischung hat eine Hydroxylzahl (mg KOH/g)von 196,7.

### Herstellung von Polyurethan-Reaktionsgemischen und Verbundfolien

Ein Polyurethan-Reaktionsgemisch wird hergestellt durch Vermischen von 50 g der Polyolmischung und der in der Tabelle 2 angegebenen Menge an Prepolymer. Diese Mengen entsprechen einer Kennzahl (NCO/OH) von ca. 140. Die Polyurethan-Reaktionsmischungen werden mit einem Holzstab intensiv für zwei Minuten gemischt und anschließend in den Walzenspalt einer Laborkaschieranlage Polytest 440 gegeben.

**Tabelle 2:**

| **Herstellung von Polyurethan-Reaktionsgemischen A - D** | | | |
|---|---|---|---|
| PUR Reaktionsgemisch für Verbund | Menge (g) des Prepolymeren | | Menge (g) der Polyolmischung |
| A | 88,3 | I | 50 |
| B | 88,0 | II | 50 |
| C | 86,9 | III | 50 |
| D | 87,8 | IV | 50 |

Die Walzentemperatur beträgt ca. 30°C, die Beschichtungsgeschwindigkeit beträgt ca. 10 m/min. Die Folienbreite beträgt 30 cm. Dabei wird folgender Folienverbund hergestellt. Die Aluminiumseite eines Polyester/Aluminium-Vorverbunds wird gegen Low Density Polyethylen (LDPE) der Type LDPE PE-K-088 (70 µ Schichtdicke), das zur besseren Verklebbarkeit auf der zu verklebenden Seite mit Korona behandelt wurde, gleitmittelhaltig verklebt. Die Auftragsmenge an Polyurethan-Reaktionsgemisch ist in Tabelle 3 angegeben.

### Prüfung der Verbundfestigkeiten

Die Auswahl der Prüfmuster erfolgt aus mindestens 20 m langen, um eine Hülse fest gewickeltem Laminat von 30 cm Bahnbreite. Die Prüfmuster werden jeweils nach Abwickeln von 5 Wickellagen aus der Mitte der Verbundfolienbahn ausgeschnitten. Vom Zeitpunkt der Herstellung an werden die Verbundfolien in einem klimatisiertem Raum bei 23°C und 50 % Luftfeuchte gelagert.

Die Prüfung der Verbundfestigkeiten erfolgen jeweils 24 h, 3 Tage, 7 Tage und 14 Tage nach Herstellung der Verbundfolien. Dazu werden je 15 mm breite Streifen der Laminate mit der Schlagschere auf ca. 30 cm Länge kantenparallel geschnitten. Die Verbundprüfung erfolgt als T-Schälung in Anlehnung an DIN 53289 mit einer VNGG-Prüfmaschine der Fa. Brugger, München, mit 100 mm/min Abzugsgeschwindigkeit auf mindestens 10 cm Länge. Die Angaben erfolgen in Newton/15 mm. Alle Ergebnisse sind Mittelwerte aus Doppelbestimmungen.

**Tabelle 3:**

| **Ergebnis der Prüfung der Verbundfestigkeiten** | | | | | | | | | |
|---|---|---|---|---|---|---|---|---|---|
| Verbund | Reaktions- gemisch nach Tabelle 2 | Auftrags-gewicht (g/ m2) | Verbundfestigkeiten in Newton /15 mm nach Tagen | | | | | | |
| | | | 1 | 2 | 3 | 7 | 14 | 21 | 28 |
| A | I | 2,8 | 5,6 | 8,8 | 10,3 | 10,8 | 10,8 | 11,8 D | 11,3 D |
| B | II | 3,0 | 5,0 | 9,2 | 10,0 | 9,2 | 11,0 | 11,6 D | 11,9 D |
| C | III | 3,0 | 4,9 | 9,8 | 10,5 | 9,7 | 11,2 D | 11,2 D | 12,3 D |
| D | IV | 3,2 | 4,5 | 7,9 | 10,2 | 10,0 | 10,2 | 10,4 D | 12,5 A |
| D = Dehnung der Folie; A = Abriss der Folie | | | | | | | | | |

### Migrationsuntersuchungen

Die Bestimmung der Migratwerte von aromatischen Polyaminen erfolgt nach der Methode, veröffentlicht in "Amtliche Sammlung von Untersuchungsverfahren nach § 35 LMBG L.00.00-6".

Der zu untersuchende Folienverbund wird auf der Hülse im Klimaraum bei 23°C und 50 % rel. Feuchte gelagert. Nach 1, 3, 7, 14 Tagen werden jeweils 10 Lagen Folienbahn abgewickelt und je zwei Folienstücke entnommen. Die Folienstücke werden so gesiegelt, dass man Beutel erhält, die eine Kontaktfläche zum Prüflebensmittel von 2 dm² haben.

Die Beutel werden mit 100 ml auf 70°C vorgewärmter 3 %-iger Essigsäure gefüllt, zugesiegelt und für 2 Stunden bei 70°C gelagert. Unmittelbar nach der Lagerung werden die Beutel entleert und die Prüflebensmittel auf Raumtemperatur abgekühlt.

Der Nachweis der migrierten Polyisocyanate erfolgt durch Diazotierung der im Prüflebensmittel vorhandenen aromatischen Amine und anschließende Kupplung mit N-(1-Naphthyl)ethylendiamin. Der gebildete Diazofarbstoff wird anschließend auf einer Trennsäule konzentriert, eluiert und im Photometer die Extinktionswerte bei 550 nm vermessen. An Hand einer Eichkurve wird in µg Aniliniumhydrochlorid/100 ml Prüflebensmittel umgerechnet. Die Nachweisgrenze wird mit 0,2 µg Aniliniumhydrochlorid/100 ml angegeben.

Die angegebenen Werte sind Mittelwerte aus drei Messungen.

**Tabelle 4:**

| **Ergebnis der Prüfung der Migratwerte** | | | | | | |
|---|---|---|---|---|---|---|
| Verbund | Gefundene Menge Amin (in µg Aniliniumhydrochlorid/100 ml) nach Tagen | | | | | |
| | 1 | 3 | 7 | 14 | 28 | 42 |
| A | 14,8 | 2,7 | 1,35 | 0,83 | 1,23 | 1,0 |
| B | 11,3 | 0,83 | <0,2 | <0,2 | <0,2 | <0,2 |
| C | 8,2 | 1,87 | 0,95 | 0,67 | 0,97 | 0,93 |
| D | 3,9 | 0,5 | <0,2 | <0,2 | <0,2 | <0,2 |

Tabelle 4 zeigt, dass in den Folienverbunden, die mit MDI-Mischungen mit hohen Gehalten an 2,4'-MDI hergestellt wurden (C und D), nach einem bzw. nach drei Tagen niedrigere Gehalte an aromatischen Aminen gefunden werden als mit den jeweils vergleichbaren (das heißt vergleichbare Gehalte an 2,2'-MDI aufweisenden) Folienverbunden, die mit MDI-Mischungen mit niedrigen Gehalten an 2,4'-MDI hergestellt wurden (A und B). Dabei ist aufgrund der vergleichbaren Gehalte an 2,2'-MDI im eingesetzten Prepolymer der direkte Vergleich der Folienverbunde A mit C sowie B mit D möglich.

Tabelle 4 zeigt weiterhin, dass die Folienverbunde, die mit MDI-Prepolyrneren mit niedrigen Gehalten an 2,2'-MDI hergestellt wurden (B und D), nach 7 Tagen weitgehend frei von aromatischen Aminen sind (unterhalb der Nachweisgrenze in diesem Test). Dagegen lassen sich in den Folienverbunden, die mit MDI-Prepolymeren mit hohen Gehalten an 2,2'-MDI hergestellt wurden (A und C), auch noch nach 42 Tagen aromatische Amine oberhalb der Nachweisgrenze finden.

Dadurch wird deutlich, dass die nach dem erfindungsgemäßen Verfahren hergestellten Fraktionen von Diisocyanaten der Diphenylmethanreihe (die den Verbundfolien B und D zugrunde liegenden MDI-Fraktionen) mit niedrigen Gehalten an 2,2'-MDI in der Anwendung zu überlegenen Produkteigenschaften im Vergleich zu den nach dem Stand der Technik hergestellten Diisocyanaten der Diphenylmethanreihe (die den Verbundfolien A und C zugrunde liegenden MDI-Fraktionen) mit hohen Gehalten an 2,2'-MDI führen.

## Patentansprüche

1. Verfahren zur Herstellung einer Fraktion von Diisocyanaten der Diphenylmethanreihe enthaltend mindestens 99 Gew.-% 2-Kern-Methylendiphenyldiisocyanat bezogen auf die Masse der Fraktion, bei dem man
a) Anilin und Formaldehyd in Gegenwart eines sauren Katalysators zu Di- und Polyaminen der Diphenylmethanreihe enthaltend 2-Kern-Methylendiphenyldiamin umsetzt, und
b) die Di- und Polyamine der Diphenylmethanreihe enthaltend 2-Kern-Methylendiphenyldiamin gegebenenfalls in Gegenwart eines Lösungsmittels phosgeniert, wobei man ein rohes Di- und Polyisocyanat erhält, und
c) aus dem rohen Di- und Polyisocyanat eine Fraktion enthaltend mindestens 95 Gew.-% 2-Kern-Methylendiphenyldiisocyanat mit einem Gehalt an 4,4'-MDI von 49 bis 95,99 Gew.-%, einem Gehalt an 2,4'-MDI von 4 bis 45 Gew.-% und einem Gehalt an 2,2'-MDI von 0,01 bis 20 Gew.-%, bezogen auf die Masse der Fraktion, abtrennt, und
d) gegebenenfalls aus der in Schritt c) erhaltenen Fraktion 4,4'-MDI zu 10 bis 98 % entfernt, und
e) aus der in Schritt c) oder in Schritt d) erhaltenen Fraktion 2,2'-MDI ganz oder teilweise abtrennt, wobei man eine Fraktion enthaltend 0 bis 0,4 Gew.-% 2,2'-MDI, 1 bis 95 Gew.-% 4,4'-MDI und 5 bis 98,6 Gew.-% 2,4'-MDI, bezogen auf die Masse der MDI-Isomeren, erhält.

2. Verfahren nach Anspruch 1, bei dem man
f) aus der Fraktion enthaltend 0 bis 0,4 Gew.-% 2,2'-MDI, 1 bis 95 Gew.-% 4,4'-MDI und 5 bis 98,6 Gew.-% 2,4'-MDI, bezogen auf die Masse der MDI-Isomeren, eine Fraktion enthaltend mindestens 99 Gew.-% 2-Kern-Methylendiphenyldiisocyanat bezogen auf die Masse der Fraktion und 0 bis 0,5 Gew.-% 2,2'-MDI, 0,1 bis 80 Gew.-% 4,4'-MDI und 20 bis 99,9 Gew.-% 2,4'-MDI, bezogen auf die Masse der MDI-Isomeren, abtrennt.

3. Verfahren nach Anspruch 1 oder 2, bei dem man in Schritt c) eine Fraktion enthaltend mindestens 98 Gew.-% 2-Kern-Methylendiphenyldiisocyanat mit einem Gehalt an 4,4'-MDI von 80 bis 93,9 Gew.-%, einem Gehalt an 2,4'-MDI von 6 bis 19,9 Gew.-% und einem Gehalt an 2,2'-MDI von 0,1 bis 5 Gew.-%, bezogen auf die Masse der Fraktion abtrennt.

4. Verfahren nach Anspruch 1, bei dem man in Schritt d) das 4,4'-MDI in einem Destillationsschritt oder einem Kristallisationsschritt abtrennt.

5. Verfahren nach Anspruch 4, bei dem man in Schritt d) 50 bis 95 % des 4,4'-MDI abtrennt.

6. Verfahren nach einem der Ansprüche 1 bis 5, bei dem man das in Schritt e) abgetrennte 2,2'-MDI per Kristallisation oder Destillation von Leichtsiedern reinigt, wobei man ein Isomerengemisch enthaltend 5 bis 99,99 Gew.-% 2,2'-MDI, 0 bis 50 Gew.-% 4,4'-MDI und 0,01 bis 95 Gew.-% 2,4'-MDI bezogen auf die Masse der MDI-Isomere erhält.

7. Verfahren nach Anspruch 6, bei dem man das Isomerengemisch enthaltend 5 bis 99,99 Gew.-% 2,2'-MDI, 0 bis 50 Gew.-% 4,4'-MDI und 0,01 bis 95 Gew.-% 2,4'-MDI bezogen auf die Masse der MDI-Isomere gegebenenfalls mit anderen Isocyanat enthaltenden Fraktionen abmischt und anschließend mit Polyethern oder Polyestern zu Polyurethanen oder Prepolymeren umsetzt

8. Verfahren nach einem der Ansprüche 1 bis 7, bei dem man in Schritt e) 50 bis 99,9999 % des mit dem rohen Di- und Polyisocyanat in die Trennung in Schritt c) eingebrachten 2,2'-MDI abtrennt.

9. Verfahren nach einem der Ansprüche 1 bis 8, bei dem man in Schritt e) eine Fraktion enthaltend 0 bis 0,3 Gew.-% 2,2'-MDI, 5 bis 85 Gew.-% 4,4'-MDI und 15 bis 95 Gew.-% 2,4'-MDI, bezogen auf die Masse der MDI-Isomeren, erhält.

10. Verfahren nach Anspruch 2, bei dem man die Destillation in Schritt f) bei 0,5 bis 30 mbar, bevorzugt bei 1 bis 15 mbar absolutem Druck durchführt.

11. Verfahren nach einem der Ansprüche 2 bis 10, bei dem man in Schritt f) eine Fraktion enthaltend 0 bis 0,35 Gew.-% 2,2'-MDI, 0,2 bis 60 Gew.-% 4,4'-MDI und 40 bis 99,8 Gew.-% 2,4'-MDI, bezogen auf die Masse der MDI-Isomeren erhält.

12. Verfahren nach einem der Ansprüche 2 bis 10, bei dem man in Schritt f) eine Fraktion enthaltend 0 bis 0,2 Gew.-% 2,2'-MDI, 0,5 bis 55 Gew.-% 4,4'-MDI und 45 bis 99,5 Gew.-% 2,4'-MDI, bezogen auf die Masse der MDI-Isomeren erhält.

13. Verfahren nach einem der Ansprüche 1 bis 12, bei dem man die Schritte d) und e) gleichzeitig in einem gemeinsamen Destillationsschritt, bevorzugt in einer Seitenstromkolonne, durchführt.

14. Verfahren nach einem der Ansprüche 2 bis 12, bei dem man die Schritte e) und f) gleichzeitig in einem gemeinsamen Destillationsschritt, bevorzugt in einer Seitenstromkolonne, durchführt.

15. Verfahren nach Anspruch 6, bei dem man die Schritte f) und die Abtrennung der Leichtsieder aus dem in Schritt e) abgetrennten 2,2'-MDI gleichzeitig in einem gemeinsamen Destillationsschritt, bevorzugt in einer Seitenstromkolonne, durchführt.

16. Verfahren zur Herstellung von Polyurethanen und Prepolymeren, bei dem man eine Fraktion enthaltend 0 bis 0,5 Gew.-% 2,2'-MDI, 0,1 bis 80 Gew.-% 4,4'-MDI und 20 bis 99,9 Gew.-% 2,4'-MDI, bezogen auf die MDI-Isomeren, nach einem der Ansprüche 1 bis 15 herstellt, und diese Fraktion anschließend mit Polyethern oder Polyestern umsetzt.
